# EUROPEAN PATENT APPLICATION

(11) **EP 4 202 532 A1**
(43) Date of publication of application: **28.06.2023**
(21) Application number: 21911300.8
(22) Date of filing: 01.12.2021
(51) Int. Cl.: G02B 27/01, G02B 27/00

(54) **ELECTRONIC APPARATUS INCLUDING SENSOR ARRAY, AND CONTROL METHOD THEREFOR**

(30) Priority: 23.12.2020 KR 20200182386
(71) Applicant: Samsung Electronics Co., Ltd., Suwon-si, Gyeonggi-do 16677 (KR)
(72) Inventor: AHN, Joongwoo, Suwon-si Gyeonggi-do 16677 (KR); KIM, Taehyeon, Suwon-si Gyeonggi-do 16677 (KR); HWANG, Minkyung, Suwon-si Gyeonggi-do 16677 (KR)
(74) Representative: HGF
(86) International application number: PCT/KR2021/018039
(87) International publication number: WO 2022/139233

(57) **Abstract**

This wearable device comprises: a housing; a plurality of optical sensors constituting at least one array and disposed on the housing or disposed so as to be exposed to the outside through the housing; and at least one processor. The at least one processor can be set to: acquire a plurality of biosignals on the basis of outputting light through the plurality of optical sensors; check the quality of the plurality of acquired biosignals; determine, on the basis of the checked quality, at least one optical sensor among the plurality of optical sensors as a sensor for identifying biological information; identify the biological information on the basis of the biosignals acquired from the at least one optical sensor; and output the identified biological information.

## Description

### [Technical Field]

The disclosure relates to an electronic device including a sensor array and a method for controlling the same.

### [Background Art]

There has been development of electronic devices including sensors capable of measuring users' biometric information. For example, an electronic device which has a sensor for measuring a user's biometric information, and which can be worn on the user's body (hereinafter, referred to as a wearable device), is being developed. A user who wears a wearable device may measure body-related information (hereinafter, referred to as biometric information) and may recognize his/her physical condition. Wearable devices may be implemented to be wearable on users' bodies in various types including, for example, a glass-type, a watch-type, a patch-type, a ring-type, or other various types.

A wearable device may use sensors so as to measure various pieces of biometric information, such as the user's heartbeat (or pulse rate), blood oxygen saturation, stress, and blood pressure. Biometric information as used herein may also be called health information or other terms. A wearable device may be capable of detecting (or sensing) a part of the user's body by using sensors. An electronic device may measure the user's various pieces of biometric information by using signals (for example, biometric signals) acquired through sensors. For example, in connection with measuring various pieces of biometric information, a wearable device may use a photo-sensor (for example, PPG sensor) in order to acquire a photoplethysmogram (PPG) signal. After light that has been output through a light emitter of an photo-sensor is projected onto the user's tissues and blood vessels, light which is reflected thereby or which has passed therethrough may be measured by a photo-detector of the photo-sensor, thereby acquiring a PPT signal, and a change in the amount of blood streams resulting from a pulse wave may be identified as the PPG signal is acquired.

### [Disclosure]

### [Technical Problem]

In order to improve the PPG signal related measurement accuracy of a wearable device, the wearable device needs to be positioned close to (for example, contact) the user's body. However, the part of the user's body contacted by the wearable device may vary depending on the user's physical size (for example, skull) or hairstyle. For example, if the photo-sensor of the wearable device is placed on a side of the user's head, the contacted part may vary depending on each user's physical size (for example, skull), and hair may come between the photo-sensor and the user's head, depending on the use's hairstyle. Light from the photo-sensor may fail to be emitted to the body as desired, or reflected or transmitted light may fail to be measured accurately because each user has a different body part contacted by the wearable device, and hair may come between the wearable device and the body part.

Various embodiments may provide an electronic device and a method for controlling the same, wherein the electronic device includes a plurality of photo-sensors, and an photo-sensor appropriate for a user who wears a wearable device (hereinafter, referred to as a wearer) is identified such that the photo-sensor appropriate for the wearer is used to identify the wearer's biometric information.

Various embodiments may provide an electronic device and a method for controlling the same, wherein the electronic device includes a plurality of sensors disposed to face different directions such that the wear's biometric signals can be measured in various directions.

### [Technical Solution]

According to various embodiments, a wearable device may include a housing, a plurality of photo-sensors that are arranged on the housing or arranged to be exposed outside through the housing, and configure at least one array, and at least one processor, wherein the at least one processor is configured to obtain a plurality of biometric signals, based on outputting light through the plurality of photo-sensors, identify qualities of the obtained a plurality of biometric signals, determine, based on the identified qualities, at least one photo-sensor among the plurality of photo-sensors as a sensor for identification of biometric information, and identify biometric information, based on a biometric signal obtained from the at least one photo-sensor, and output the identified biometric information.

According to various embodiments,, a method for controlling a wearable device may include obtaining a plurality of biometric signals, based on outputting light through a plurality of photo-sensors of the wearable device, the plurality of photo-sensors being arranged on a housing of the wearable device or arranged to be exposed outside through the housing, identifying qualities of the obtained a plurality of biometric signals, determining, based on the identified qualities, at least one photo-sensor among the plurality of photo-sensors as a sensor for identification of biometric information, and identifying biometric information, based on a biometric signal obtained from the at least one photo-sensor, and outputting the identified biometric information.

According to various embodiments,, a wearable device may include a housing, a plurality of photo-sensors arranged to be exposed outside through the housing, the plurality of photo-sensors configuring a first array disposed to face a first direction, and a second array disposed to face a second direction different from the first direction, and at least one processor, wherein the at least one processor is configured to obtain a plurality of first biometric signals, based on outputting first light through at least one first of photo-sensors configuring the first array, obtain a plurality of second biometric signals, based on outputting second light through at least some of photo-sensors configuring the second array, apply different weight values to the obtained a plurality of first biometric signals and the obtained a plurality of second biometric signals so as to identify biometric information, and output the identified biometric information.

### [Advantageous Effects]

According to various embodiments, a wearable device may identify an photo-sensor appropriate for a wearer and may identify the wearer's biometric information by using the photo-sensor appropriate for the wearer, thereby providing more accurate biometric information.

According to various embodiments, a wearable device may measure the wearer's biometric signals in various directions, thereby providing more accurate biometric information.

Various advantages effects provided by the disclosure are not limited to the above-mentioned advantageous effects.

### [Description of the Drawings]

FIG. 1 is a block diagram illustrating an electronic device in a network environment according to various embodiments;
FIG. 2A is a block diagram illustrating elements of a wearable device according to various embodiments;
FIG. 2B is a block diagram illustrating operations of a wearable device and an external electronic device according to various embodiments;
FIG. 3 illustrates an example of a wearable device according to various embodiments;
FIG. 4 illustrates an example of a photo-sensor according to various embodiments;
FIG. 5A illustrates an example of a photo-sensor array including a plurality of photo-sensors according to various embodiments;
FIG. 5B illustrates an example of a photo-sensor array including a plurality of photo-sensors according to various embodiments;
FIG. 5C is a diagram illustrating an arrangement of a plurality of photo-sensor arrays according to various embodiments;
FIG. 5D is a diagram illustrating an arrangement of a plurality of photo-sensor arrays according to various embodiments;
FIG. 6 is a flowchart of a method of determining, by a wearable device, a photo-sensor for identification of biometric information according to various embodiments;
FIG. 7 is a flowchart of a method of identifying, by a wearable device, biometric information, based on a weight value according to various embodiments;
FIG. 8 is a flowchart of a method of storing, by a wearable device, information on a photo-sensor determined as a sensor for identification of biometric information according to various embodiments;
FIG. 9 is a flowchart of an operation related to whether a photo-sensor for identification of biometric information is determined by a wearable device, according to various embodiments;
FIG. 10 is a flowchart of a method of re-determining, by a wearable device, a photo-sensor for identification of biometric information according to various embodiments;
FIG. 11 is a flowchart of a method of applying, by a wearable device, a weight value to a plurality of photo-sensor arrays so as to identify biometric information according to various embodiments; and
FIG. 12 illustrates an example of a screen showing biometric information which may be displayed through a display of a wearable device according to various embodiments.

### [Mode for Invention]

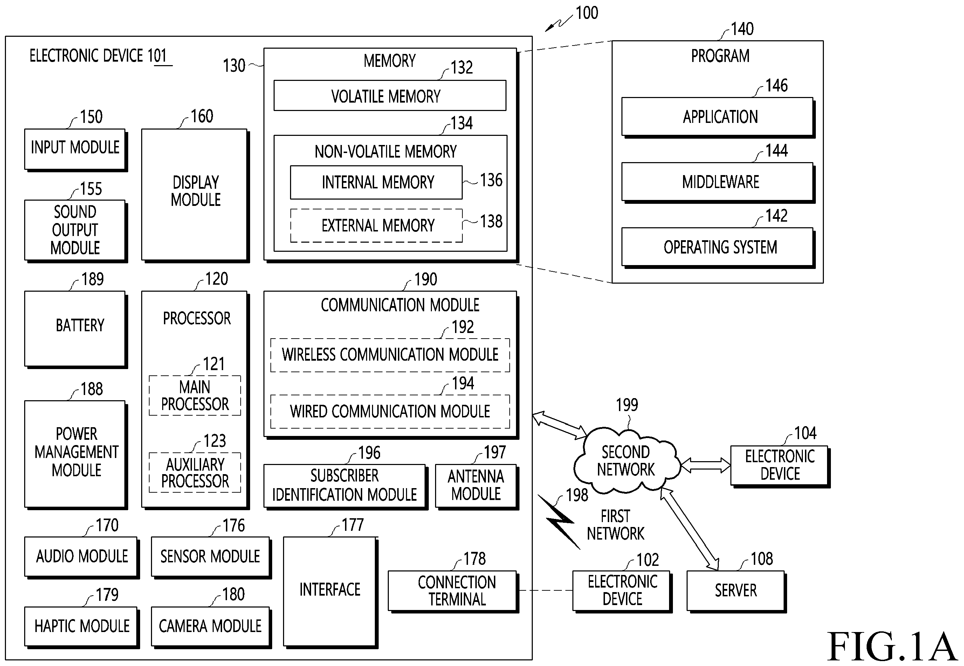

FIG. 1 is a block diagram illustrating an electronic device in a network environment according to various embodiments.

Referring to FIG. 1, an electronic device 101 in a network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, a memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control, for example, at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active (e.g., executing an application) state. According to an embodiment, the auxiliary processor 123 (e.g., an image signal processor or a communication processor) may be implemented as part of another component (e.g., the camera module 180 or the communication module 190) functionally related to the auxiliary processor 123. According to an, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or an external electronic device (e.g., an electronic device 102 (e.g., a speaker or a headphone)) directly or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive a plurality of-input and a plurality of-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

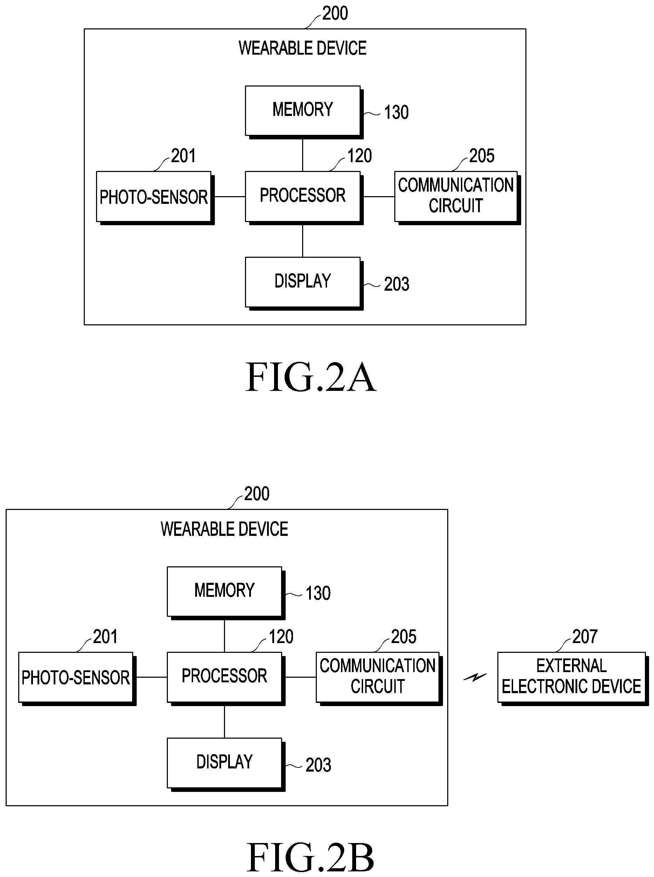

FIG. 2A is a block diagram illustrating elements of a wearable device according to various embodiments.

According to various embodiments, a wearable device 200 may include at least one of a photo-sensor 201 (e.g., the sensor module 176 in FIG. 1), a memory 130, a processor 120, a display 203 (e.g., the display module 160 in FIG. 1), or a communication circuit (e.g., the communication module 190 in FIG. 1).

According to various embodiments, the photo-sensor 201 may include a light emitting unit and a light receiving unit. For example, the light emitting unit may include at least one of a spectrometer, a vertical cavity surface emitting laser (VCSEL), a light emitting diode (LED), a white LED, or a white laser. For example, the light receiving unit may include at least one of an avalanche photodiode (PD), a single-photon avalanche diode (SPAD), a photodiode, a photomultiplier tube (PMT), a charge coupled device (CCD), a CMOS array, or a spectrometer. According to various embodiments, a structure of the light receiving unit may have a reflective type or a transmissive type. However, the configuration included in the photo-sensor 201 is not limited to the light emitting unit and the light receiving unit. For example, the photo-sensor 201 may further include a signal processor (not illustrated) (e.g., an analog front end). The signal processor (not illustrated) may include an amplifier configured to amplify a biometric signal, and an analog-to-digital converter (ADC) configured to convert an analog biometric signal into a digital biometric signal. However, the configuration included in the signal processor 133 is not limited to the amplifier and the ADC described above. According to various embodiments, the photo-sensor 201 may be a PPG sensor or a laser diode (LD) and an image sensor, and may include various other types of sensors that output light to the outside and receive light from the outside. According to various embodiments, the photo-sensor 201 may output light to the outside through the light emitting unit. For example, the light emitting unit may output at least one of an infrared ray, red light, green light, or blue light, and may include a light emitting element (e.g., an LED) corresponding to each of at least one output light. According to various embodiments, light output by the photo-sensor 201 to the outside is emitted to a wearer's body, and at least a part of the emitted light may be reflected by the user's body (e.g., skin, skin tissue, a fat layer, a vein, an artery, or a capillary vessel). According to various embodiments, the photo-sensor 201 may receive, through the light receiving unit, light obtained through reflection by a wearer's body, and may output an electrical signal (hereinafter, a biometric signal) corresponding to the received light to at least one hardware element (e.g., the processor 120) of the wearable device 200. According to various embodiments, the wearable device 200 may include a plurality of photo-sensors 201, and the plurality of photo-sensors may configure at least one array. According to various embodiments, different weight values may be applied to the plurality of photo-sensors (or biometric signals obtained from the plurality of photo-sensors). According to various embodiments , the photo-sensor 201 may be disposed on a housing of the wearable device 200, or may be disposed to be exposed outside through the housing. The number and the arrangement positions or directions of photo-sensors 201 will be described in more detail with reference to the drawings described below.

According to various embodiments, the memory 130 may store various pieces of information. For example, data (e.g., data on the current value, the voltage value, the intensity, or the quality (e.g., a signal-to-noise ratio (SNR)) of a biometric signal) on a biometric signal of a wearer may be stored in the memory 130. For example, biometric information (e.g., information on a heart rate (or a pulse rate), an oxygen saturation level of blood, stress, or a blood pressure) of a wearer may be stored in the memory 130. For example, information (e.g., identification information) on a sensor to be used for identification of biometric information may be stored in the memory 130. For example, information on a weight value to be applied during identification of biometric information may be stored in the memory 130. According to various embodiments, the information (e.g., identification information) on a sensor to be used for identification of biometric information, or the information on a weight value to be applied during identification of biometric information, which are described above, may be stored depending on a wearer (e.g., in association with identification information of the wearer). The above examples correspond to examples of pieces of information which may be stored in the memory 130, and various pieces of information which may be used in the operation of the wearable device 200 may be stored in the memory 130.

According to various embodiments, the processor 120 may perform and/or control overall operations of the wearable device 200. For example, the processor 120 may perform a designated operation of the wearable device 200, or may control another hardware element (e.g., the memory 130, the photo-sensor 201, the display 203, or the communication circuit 205) to perform a designated operation.

According to various embodiments, the processor 120 may obtain (or receive) a biometric signal output by the photo-sensor 201. According to various embodiments, the processor 120 may identify a quality (e.g., SNR) of the obtained biometric signal. According to various embodiments, the processor 120 may identify biometric information (e.g., information on a heart rate (or a pulse rate), an oxygen saturation level of blood, stress, or a blood pressure) of a wearer, based on the obtained biometric signal. According to various embodiments, the wearable device 200 may further include a sensor (e.g., an acceleration sensor) different from the photo-sensor 201, and the processor 120 may also identify biometric information of a wearer, based on a biometric signal obtained from the photo-sensor 201, and sensing data obtained from a sensor (not illustrated) (e.g., an acceleration sensor) different from the photo-sensor 201.

According to various embodiments, in a case where the wearable device 200 includes a plurality of photo-sensors 201, the processor 120 may determine at least one photo-sensor for identification of biometric information among the plurality of photo-sensors. For example, the processor 120 may obtain biometric signals from the plurality of photo-sensors, respectively, and compare qualities of the obtained biometric signals so as to determine at least one photo-sensor for identification of biometric information. This process will be described in more detail with reference to the drawings described below. According to various embodiments, the processor 120 may store information (e.g., identification information) on the determined at least one photo-sensor in the memory 130.

According to various embodiments, the processor 120 may identify biometric information of a wearer by using a biometric signal obtained from the at least one photo-sensor determined for identification of biometric information. For example, the processor 120 may activate only the determined at least one photo-sensor among the plurality of photo-sensors, and identify biometric information of a wearer by using a biometric signal obtained from the at least one photo-sensor. More specifically, the processor 120 may control a light emitting unit corresponding to the determined at least one photo-sensor among light emitting units corresponding to the plurality of photo-sensors such that the light emitting unit outputs light, and thus obtain a biometric signal corresponding to light (or light received by a light receiving unit corresponding to the determined at least one photo-sensor) received by light receiving units corresponding to the plurality of photo-sensors. Alternatively, the processor 120 may control a light receiving unit corresponding to the determined at least one photo-sensor among the light receiving units corresponding to the plurality of photo-sensors such that the light receiving unit receives light from the outside, or control the light receiving unit corresponding to the determined at least one photo-sensor to output a biometric signal corresponding to the received light so as to obtain a biometric signal from the at least one photo-sensor. As another example, the processor 120 may control the entirety or some of the plurality of photo-sensors to output light, obtain biometric signals from the entirety or some of the plurality of photo-sensors, then apply a high weight value to a biometric signal obtained from the determined at least one photo-sensor, and apply a low weight value to biometric signals obtained from the other photo-sensors so as to identify biometric information of a wearer.

According to various embodiments, the processor 120 may store, in the memory 130, information related to an operation of obtaining a biometric signal of a wearer or an operation of identifying biometric information. For example, the processor 120 may store, in the memory 130, at least one of data (e.g., data on the current value, the voltage value, the intensity, or the quality (e.g., a signal-to-noise ratio (SNR)) of a biometric signal) on a biometric signal of a wearer, or biometric information (e.g., information on a heart rate (or a pulse rate), an oxygen saturation level of blood, stress, or a blood pressure) of a wearer. For example, the processor 120 may store, in the memory 130, information on a weight value to be applied to biometric signals (or photo-sensors) during identification of biometric information.

According to various embodiments, the processor 120 may control the display 203 to display identified biometric information of a wearer. For example, the processor 120 may display information (e.g., a numerical value) on a heart rate (or a pulse rate), an oxygen saturation level of blood, stress, or a blood pressure of the wearer. According to various embodiments, the processor 120 may display various information which can be predicted from the identified biometric information. For example, the processor 120 may display, in a text or image type, information relating to whether the wearer has a health problem, which is predicted from the identified biometric information. According to various embodiments, the processor 120 may also output the identified biometric information of the wearer through other output devices (e.g., a speaker) in various types (e.g., voice).

According to various embodiments, the communication circuit 205 may transmit data to an external electronic device (e.g., the external electronic device 207 in FIG. 2B) by wire or wirelessly, or receive data from an external electronic device (e.g., the external electronic device 207 in FIG. 2B), and this process will be described in more detail with reference to the drawings described below.

FIG. 2B is a block diagram illustrating operations of a wearable device and an external electronic device according to various embodiments. A description overlapping with the above description given with reference to the drawings will be omitted.

According to various embodiments, an external electronic device 207 may include at least a part of the elements of the electronic device 101 in FIG. 1. For example, the external electronic device 207 may include a display module, a communication module, and/or a memory.

According to various embodiments, the wearable device 200 and the external electronic device 207 may transmit and/or receive data. For example, data transmission and/or reception between the wearable device 200 and the external electronic device 207 may be performed by wire or wirelessly. For example, the wearable device 200 and the external electronic device 207 may be connected through a first network (e.g., the first network 198 in FIG. 1) or a second network (e.g., the second network 199 in FIG. 1).

According to various embodiments, according to data transmission and/or reception between the wearable device 200 and the external electronic device 207, at least a part of the operations of the wearable device 200 described in the disclosure may be performed by the external electronic device 207.

For example, the external electronic device 207 may receive, from the wearable device 200, data (e.g., data on the current value, the voltage value, the intensity, or the quality (e.g., an SNR) of a biometric signal) on a biometric signal, and identify biometric information (e.g., information on a heart rate (or a pulse rate), an oxygen saturation level of blood, stress, or a blood pressure) of the wearer. The external electronic device 207 may transmit the identified biometric information to the wearable device 200, and a screen relating to the biometric information may be displayed through the wearable device 200.

For example, the external electronic device 207 may display biometric information of a wearer through a display (not illustrated) of the external electronic device 207. The external electronic device 207 may display, through the display (not illustrated), a wearer's biometric information that is received from the wearable device 200 or is identified based on data on a biometric signal, which is received from the wearable device 200.

For example, the external electronic device 207 may store, in a memory (not illustrated) of the external electronic device 207, data (e.g., data on the current value, the voltage value, the intensity, or the quality (e.g., an SNR) of a biometric signal) on a biometric signal, or a wearer's biometric information. The external electronic device 207 may store data on a biometric signal, which is received from the wearable device 200 in the memory (not illustrated). The external electronic device 207 may store, in the memory (not illustrated), a wearer's biometric information that is received from the wearable device 200 or is identified based on data on a biometric signal, which is received from the wearable device 200. The external electronic device 207 may store the identified biometric information, depending on a wearer (e.g., store the information to correspond to identification information of a wearer).

For example, the external electronic device 207 may determine at least one photo-sensor for identification of biometric information among a plurality of photo-sensors of the wearable device 200. The external electronic device 207 may determine at least one photo-sensor for identification of biometric information among the plurality of photo-sensors of the wearable device 200, based on data on a biometric signal, which is received from the wearable device 200. The external electronic device 207 may store information (e.g., identification information) on at least one photo-sensor for identification of biometric information, or transmit same to the wearable device 200. The external electronic device 207 may store, in the memory (not illustrated), information on at least one photo-sensor determined based on information on a biometric signal, which is received from the wearable device 200, or may store, in the memory (not illustrated), information on at least one photo-sensor for identification of biometric information, which is received from the wearable device 200. The external electronic device 207 may transmit, to the wearable device 200, information on at least one photo-sensor determined based on information on a biometric signal, which is received from the wearable device 200. The external electronic device 207 may determine weight values to be applied to the plurality of photo-sensors, or may receive information on a weight value, and then store the information on the weight value in the memory (not illustrated), or transmit same to the wearable device 200. The external electronic device 207 may store information (e.g., identification information) on the determined at least one photo-sensor, or information on weight values for the plurality of photo-sensors according to a wearer (e.g., store the information to correspond to identification information of the wearer).

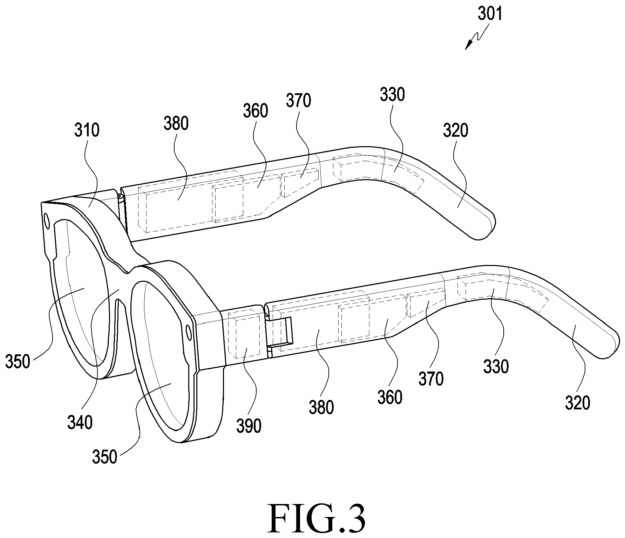

FIG. 3 illustrates an example of a wearable device according to various embodiments.

Referring to FIG. 3, a wearable device (e.g., the wearable device 200 in FIG. 2A) may be glasses type augmented reality (AR) glasses 301 which are wearable on a user's head.

According to various embodiments, the AR glasses 301 may include a pair of display devices 350 and a pair of housings 310. According to various embodiments, the pair of display devices 350 may be fixed to the pair of housings 310 each having a frame shape, respectively. According to various embodiments, a pair of wearing members 320 may extend from the pair of housings 310 so as to be parallel to each other.

According to various embodiments, the AR glasses 301 may include a gap adjustment structure 340 configured to adjust the length between the pair of housings 310, and a circuit substrate 360, a battery 370, and a photo-sensor array 330 arranged in the wearing member 320. A light output device 380 (e.g., a projector), a light refracting module 390 (e.g., a prism), or a display module (not illustrated) may be included in the wearing member 320 of the electronic device 101.

According to various embodiments, the display device 350 may include a display module, a projector, or a sensor having a touch circuit mounted therein, and a display (e.g., the display 203 in FIG. 2A) of the display device 350 may be a transparent or translucent display. For example, the display device 350 may include a window member (e.g., a transparent member), and the window member may include a light adjustment member disposed at least a part thereof. The light adjustment member may be a glass made of a translucent material, or may be a member having a light transmissivity which can be adjusted according to adjustment of a pigmentation level. For example, the display device 350 may include a lens including a waveguide, or a reflective lens, and each lens may transfer light output from the output device to a user's eyes.

According to various embodiments, the pair of housings 310 may have frame shapes at least partially surrounding the edges of the display devices 350, respectively, and may function as rims of a glasses structure including general sunglasses.

According to various embodiments, the circuit substrates 360 may be arranged in the pair of wearing members 320, respectively, and a circuit wire connecting the circuit substrates 360 may be disposed inside or outside the pair of housings 310. According to various embodiments, the pair of wearing members 320 may function as the temples of a general glasses structure. For example, the pair of housings 310 may be positioned on a user's face so as to enable the display devices 350 to be positioned to correspond to the user's eyes, and the pair of wearing members 320 may be stably placed on the user's ears at both sides of the user's head, respectively.

According to various embodiments, the pair of wearing members 320 may be used for arrangement of the circuit substrate 360, the battery 370, the light output device 380, the photo-sensor array 330, and the light refracting module 390 of the AR glasses 301. For example, each of the pair of wearing members 320 may be provided with a housing structure capable of accommodating the circuit substrate 360, the battery 370, the photo-sensor array 330, the light output device 380, or the light refracting module 390. As another example, the AR glasses 301 may include the circuit substrates 360, the batteries 370, the photo-sensor arrays 330, the light output devices 380, and the light refracting modules 390 in the pair of wearing members 320, respectively. As another example, the arrangement of the circuit substrate 360, the battery 370, the photo-sensor array 330, the light output device 380, or the light refracting module 390 may be variously changed based on the weight distribution and the wearing comfort of the AR glasses 301.

According to various embodiments, a plurality of circuit substrates 360 may be configured, and one of the circuit substrates may be provided as a substrate including at least one of a driving circuit of the display device 350, a processor (e.g., the processor 120 in FIG. 1) for processing of image information, or a communication circuit (e.g., the communication circuit 205 in FIG. 2A) for communication with an external electronic device (e.g., the external electronic device 207 in FIG. 2B). According to various embodiments, another one of the circuit substrates 360 may be provided as a circuit substrate in which at least one of an interface for a user, various types of connectors and a communication module configured to provide access of a commercial communication network or another electronic device, or a sensor module is mounted. According to various embodiments, another one of the circuit substrates 360 may be provided as a substrate including at least one of a microphone, a speakerphone for input or output of sound, or one of the circuit substrates 360, and may be disposed to be adjacent to one of the circuit substrates 360. However, the circuit arrangement of the circuit substrates 360, and the function thereof are not limited thereto, and can be variously adjusted as necessary. According to various embodiments, the circuit substrates 360 may be arranged in one of the wearing members 320.

According to various embodiments, a sensor module provided in the circuit substrates 360 may include a proximity sensor, an illuminance sensor, a gyro sensor, a camera module, an eye tracker, a geomagnetic sensor, or an acceleration meter, and various types of sensors included in the sensor module are not necessarily required to be arranged in one of the circuit substrates 360. For example, the camera module may be disposed at a proper position on the pair of housings 310 so as to be adjacent to a user's gaze.

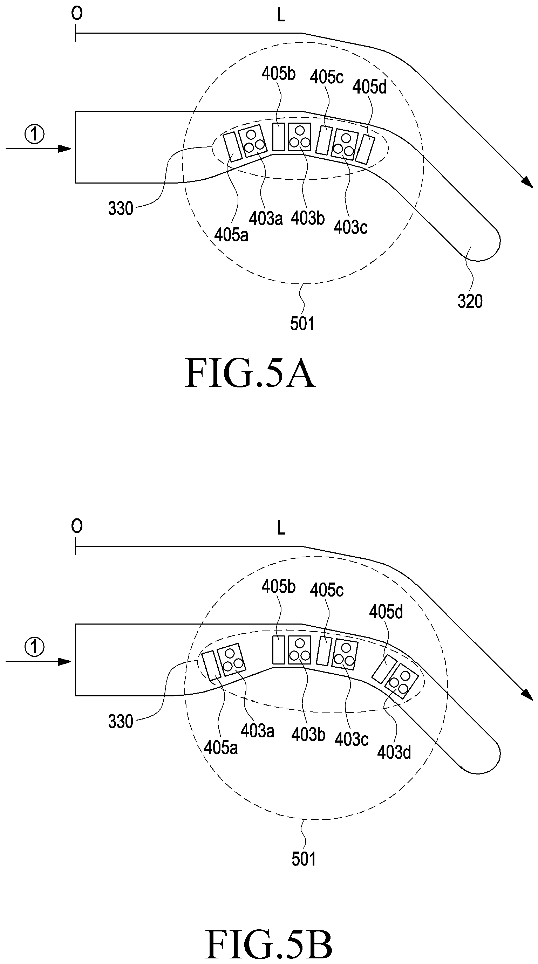

According to various embodiments, the photo-sensor array 330 may be disposed in one of the pair of wearing members 320, or may be disposed in each of the pair of wearing members 320. According to various embodiments, the photo-sensor array 330 may be disposed on the outer surface of one of the pair of wearing members 320, or may be disposed on the outer surface of each of the pair of wearing members 320. According to various embodiments, the photo-sensor array 330 may include a plurality of photo-sensors (e.g., the photo-sensor 201 in FIG. 2A), and may be disposed at a proper position in the pair of wearing members 320. For example, the photo-sensor array 330 may be disposed near a curved part (e.g., the part 501 in FIG. 5A) (e.g., a part curved to be disposed on a wearer's ear) so as to be less affected by a body difference (e.g., a head shape difference, or a hair position difference) between wearers and so as to come into close contact with a wearer's body, and this disposition will be described in more detail with reference to the drawings described below.

According to various embodiments, one or more batteries 370 may be configured, and may be arranged in at least one of the pair of wearing members 320 or arranged in each of the wearing members 320, so as to provide power to the circuit substrate 360, the photo-sensor array 330, or the display device 350.

According to various embodiments, a plurality of light output devices 380 and a plurality of light refracting modules 390 may be arranged, and may be positioned in at least one of the pair of wearing members 320 or arranged in each of the wearing members 320. Light output from the light output device 380 may pass through the light refracting module 390 and then reach the display device 350. The AR glasses 301 using the light output device 380 may have a waveguide type or a reflective mirror type. For example, in the waveguide type, light emitted from a side light output device, such as a projector, is reflected by a grating area formed on a display device by means of a waveguide, such as a prism, and then the reflected light may be transferred to a user's eye. For example, in the reflective mirror type, light output from a light output device is directly reflected by a display device in front of a user's eye, whereby visual information can be provided to the user's eye.

According to various embodiments, the circuit substrates 360 arranged in each of the pair of housings 310 may be connected to each other by a circuit wire (not illustrated). The circuit wire may provide a transmission/reception path of various types of control signals and data between the circuit substrates. The circuit wire may be configured by using a coaxial cable, and may have various types of transmission line structures, such as a flexible printed circuit board (FPCB).

According to various embodiments, the AR glasses 301 may include an input device including a physical key or touch pad. For example, an input module, such as a power key or a touch pad, is a device requiring a user's direct contact, and may be exposed to the outside of the AR glasses 301.

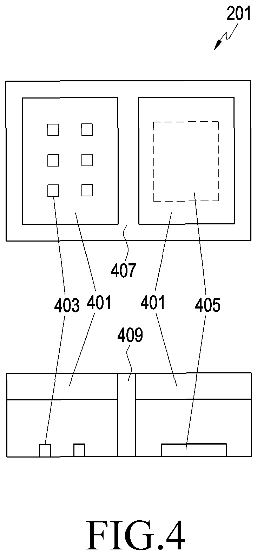

FIG. 4 illustrates an example of a photo-sensor according to various embodiments.

According to various embodiments, the photo-sensor 201 may include at least one of an optical window 401, a light emitting unit 403, a light receiving unit 405, an optical shield 407, or an optical wall (optical septum) 409.

According to various embodiments, the photo-sensor 201 may be exposed to the outside through at least a part of a housing configuring a wearable device (e.g., the wearable device 200 in FIG. 2A).

According to various embodiments, the optical window 401 may cover the light emitting unit 403 and the light receiving unit 405. According to various embodiments, light (or an optical signal) output from the light emitting unit 403 may be output to the outside through the optical window 401. According to various embodiments, light (or an optical signal) input from the outside may be received by the light receiving unit 405 through the optical window 401. According to various embodiments, the optical window 401 may be implemented using a transparent material (e.g., glass or plastic).

According to various embodiments, the light emitting unit 403 may output light (or an optical signal) to the outside. For example, the light emitting unit 403 may output light to a wearer's body (e.g., skin). For example, the light emitting unit 403 may output at least one of infrared rays, red light, green light, or blue light sequentially (e.g., time-division) or simultaneously. For example, the light emitting unit 403 may include at least one of a spectrometer, a VCSEL, an LED, a white LED, or a white laser.

According to various embodiments, the light receiving unit 405 may receive light (or an optical signal) input from the outside. For example, the light receiving unit 405 may receive at least partial light (or an optical signal), among the light output from the light emitting unit 403, which is obtained through reflection by a wearer's body (e.g., skin, skin tissue, a fat layer, a vein, an artery, or a capillary vessel). According to various embodiments, the light receiving unit 405 may output an electrical signal (e.g., a biometric signal) corresponding to received light. For example, the light receiving unit 405 may include at least one of an avalanche photodiode, a single-photon avalanche diode, a photodiode, a photomultiplier tube, a charge coupled device, a CMOS array or a spectrometer.

According to various embodiments, the light emitting unit 403 and the light receiving unit 405 may be exposed to the outside through at least a part of a housing configuring the wearable device 200.

In FIG. 4, the number, the shape, the size, and the position of the light emitting unit 403 (or an element included in the light emitting unit 403) and the light receiving unit 405 (or an element included in the light receiving unit 405) are designated and illustrated. However, the number, the shape, the size, and the position of the light emitting unit 403 and the light receiving unit 405 are not limited thereto, and may be variously implemented.

According to various embodiments, the optical shield 407 may block light (or an optical signal) input from the outside. For example, the optical shield 407 may include a material capable of blocking light input from the outside. According to various embodiments, the shape or size of the optical shield 407 may be variously configured.

According to various embodiments, the optical wall 409 may block light movement between the light emitting unit 403 and the light receiving unit 405. For example, the optical wall 409 may be positioned between the light emitting unit 403 and the light receiving unit 405. For example, the optical wall 409 may block light output from the light emitting unit 403 from being directly input to the light receiving unit 405.

According to various embodiments, the photo-sensor 201 may further include an electrode unit (not illustrated) which is exposed to the outside through at least a part of a housing configuring the wearable device 200. For example, the electrode unit (not illustrated) may be implemented using a conductive member through which current can flow. For example, the electrode unit (not illustrated) may be implemented using a conductive member (stainless steel, silver, and/or gold) having low resistance. According to various embodiments, the electrode unit (not illustrated) may include a plurality of electrodes, and the shape or size of each of the plurality of electrodes may be variously configured. According to various embodiments, the electrode unit (not illustrated) may be electrically connected to at least one biometric sensor (e.g., the sensor module 176 in FIG. 1) included in the wearable device 200, so as to be used to obtain a user's biometric information, body information, or health information. According to various embodiments, the electrode unit (not illustrated) may be used to, through a biometric sensor included in the wearable device 200, perform a bioelectric impedance analysis (BIA) measurement and measure a user's fat percentage, may be used to perform an electrocardiogram (ECG) measurement and measure a user's electrocardiogram, and may be used to perform a galvanic skin response (GSR) measurement and measure (or calculate) a user's skin resistance and/or skin moisture content.

FIG. 5A illustrates an example of a photo-sensor array including a plurality of photo-sensors according to various embodiments.

Referring to FIG. 5A, according to various embodiments, the photo-sensor array 330 may be disposed at one of the pair of wearing members 320 of the wearable device 200, or disposed at each of the wearing members 320. According to various embodiments, the photo-sensor array 330 may be configured by a plurality of photo-sensors (e.g., the photo-sensor 201 in FIG. 2A). In the disclosure, the plurality of photo-sensors (e.g., the photo-sensor 201 in FIG. 2A) configuring the photo-sensor array 330 may imply not only that the photo-sensor array 330 includes a plurality of photo-sensors (e.g., the photo-sensor 201 in FIG. 2A) including a light emitting unit (e.g., the light emitting unit 403 in FIG. 4) and a light receiving unit (e.g., the light receiving unit 405 in FIG. 4), but also that a plurality of light emitting units 403a, 403b, and 403c, and a plurality of light receiving units 405a, 405b, 405c, and 405d configure the photo-sensor array 330.

Referring to FIG. 5A, one wearing member among the pair of wearing members 320 of the wearable device 200 is illustrated. At least one photo-sensor array may be disposed at one wearing member, and FIG. 5A illustrates the wearing member 320 at which one photo-sensor array is disposed.

According to various embodiments, the plurality of light emitting units 403a, 403b, and 403c, and the plurality of light receiving units 405a, 405b, 405c, and 405d may be arranged at the wearing member 320. For example, the plurality of light emitting units 403a, 403b, and 403c, and the plurality of light receiving units 405a, 405b, 405c, and 405d may be arranged near a curved part 501 of the wearing member 320. For example, the curved part 501 may be a part which can come into contact with an upper side of a wearer's ear (e.g., an earflap) when the wearable device 200 is worn on the wearer, and may be properly curved. Depending on a wearer, a position at which the wearing member 320 and the wearer's body (e.g., an ear) come into contact with each other when the wearing member is worn may be different. For example, statistically, a contact position of the wearing member 320 may be about 6 cm to 8.5 cm in a case of a person positioned relatively close to a start position O of the wearing member 320, and may be about 8.5 cm to 11 cm in a case of a person positioned relatively far away from the start position. The plurality of light emitting units 403a, 403b, and 403c, and the plurality of light receiving units 405a, 405b, 405c, and 405d may be arranged in a section from 7 cm to 10 cm with reference to the start position O when a length (e.g., the length L of a transverse curved line) of the wearing member 320 is 13 cm. For example, the gap between one light emitting unit and one light receiving unit may be about 2 mm to 6 mm. For example, the total arrangement length of the photo-sensor array 330 configured by the plurality of light emitting units 403a, 403b, and 403c and the plurality of light receiving units 405a, 405b, 405c, and 405d may be about 30 mm. The position, gap, or arrangement length described above correspond to examples, and may be determined to be various values according to various standards.

FIG. 5B illustrates an example of a photo-sensor array including a plurality of photo-sensors according to various embodiments.

According to various embodiments, the light emitting units 403a, 403b, 403c, and 403d and the light receiving units 405a, 405b, 405c, and 405d may be arranged with different densities. In an example, with respect to the statistic described with reference to FIG. 5A, a position on the wearing member 320 with which a wearer's body (e.g., an ear) comes into contact may be about 6 cm to 8.5 cm in a case of a person positioned relatively close to the start position O, and may be about 8.5 cm to 11 cm in a case of a person positioned relatively far away from the start position. Therefore, depending on a wearer, there is a possibility that the wearer's body (e.g., an ear) comes into contact with the wearing member 320 within the range of about 6 cm to 11 cm, and generally (e.g., stochastically), there may be a high possibility that the wearer's body (e.g., an ear) comes into contact with the wearing member 320 within the range of about 7 cm to 10 cm. Therefore, light emitting units 403a, 403b, 403c, and 403d and light receiving units 405a, 405b, 405c, and 405d may be arranged within a range providing a possibility of contact of a wearer's body (e.g., an ear), for example, the range of about 6 cm to 11 cm. Light emitting units and light receiving units positioned within a range providing a high possibility of contact of a wearer's body (e.g., an ear), for example, the range of about 7 cm to 10 cm may be relatively closely arranged together (e.g., a large number of light emitting units and a large number of light receiving units are arranged). Light emitting units and light receiving units positioned within the other ranges (e.g., the range of about 6 cm to 8.5 cm and/or the range of about 10 cm to 11 cm) may be relatively scarcely (e.g., sparsely) arranged. The range described above corresponds to an example, and may be determined to be various values according to various standards.

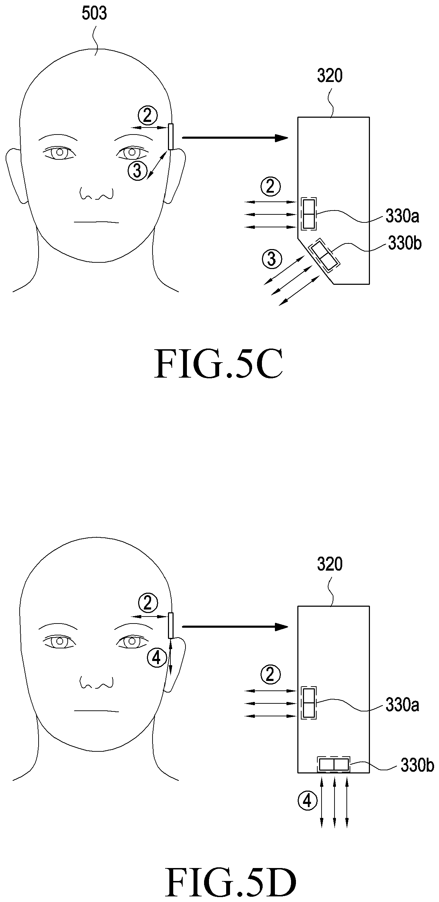

FIG. 5C is a diagram illustrating an arrangement of a plurality of photo-sensor arrays (e.g., the photo-sensor array 330 in FIG. 5A) according to various embodiments. FIG. 5D is a diagram illustrating an arrangement of a plurality of photo-sensor arrays (e.g., the photo-sensor array 330 in FIG. 5A) according to various embodiments.

FIGS. 5C and 5D illustrate a wearable device (e.g., the wearable device 200 in FIG. 2A) which is worn on a wearer 503 when viewed in a front direction (e.g., direction ① in FIG. 5A) of the wearer 503. According to various embodiments, a plurality of photo-sensor arrays 330a and 330b may be arranged at the wearing member 320 (e.g., each of the pair of wearing members). Each of the photo-sensor arrays may include a plurality of light emitting units (e.g., the light emitting units 403a, 403b, and 403c in FIG. 5A) and a plurality of light receiving units (e.g., the light receiving units 405a, 405b, 405c, and 405d in FIG. 5A).

Referring to FIG. 5C, according to various embodiments, the first photo-sensor array 330a may be disposed to face a first direction ② which is oriented toward a head side surface of the wearer 503 (e.g., which is substantially perpendicular to the head side surface), and the second photo-sensor array 330b may be disposed to face a second direction ③ which is oriented toward an ear of the wearer 503 (e.g., which is oblique to the head side surface). Generally, side hair of the wearer 503 is positioned on a head side surface of the wearer 503, and hair may be relatively less near an ear on the head side surface. Therefore, because there are less obstacles (e.g., hair) between the body (e.g., the skin) of the wearer 503 and the second photo-sensor array 330b disposed to face the second direction ③ , light output from one or more photo-sensors configuring the second photo-sensor array 330b may be relatively effectively emitted to (e.g., may reach) the body (e.g., the skin), and light obtained through reflection by the body (e.g., the skin) may be relatively effectively received by (e.g., may reach) the one or more photo-sensors configuring the second photo-sensor array 330b. Therefore, the wearable device 200 may obtain a biometric signal having a higher quality (e.g., a higher SNR) from the second photo-sensor array 330b compared to the first photo-sensor array 330a.

Referring to FIG. 5D, according to various embodiments, the first photo-sensor array 330a may be disposed to face a first direction ② which is oriented toward a head side surface of the wearer 503 (e.g., which is substantially perpendicular to the head side surface), and the second photo-sensor array 330b may be disposed to face a third direction (4) which is oriented toward an ear of the wearer 503 (e.g., which is substantially perpendicular to an upper side of the ear). Generally, side hair of the wearer 503 is positioned on a head side surface of the wearer 503, and a possibility that hair is positioned near an ear may be relatively small. Therefore, because there are less obstacles (e.g., hair) between the body (e.g., the skin) of the wearer 503 and the second photo-sensor array 330b disposed to face the third direction ④ , light output from one or more photo-sensors configuring the second photo-sensor array 330b may be relatively effectively emitted to (e.g., may reach) the body (e.g., the skin), and light obtained through reflection by the body (e.g., the skin) may be relatively effectively received by (e.g., may reach) the one or more photo-sensors configuring the second photo-sensor array 330b. Therefore, the wearable device 200 may obtain a biometric signal having a higher quality (e.g., a higher SNR) from the second photo-sensor array 330b compared to the first photo-sensor array 330a.

As described with reference to FIG. 5C or FIG. 5D, a higher quality (e.g., a higher SNR) of biometric signal can be obtained from the second photo-sensor array 330b disposed to face the second direction ③ or the third direction (4) than from the first photo-sensor array 330a disposed to face the first direction ②. Therefore, the wearable device 200 (e.g., the processor 120 in FIG. 1) may apply a higher weight value to the second photo-sensor array 330b than to the first photo-sensor array 330a. For example, the wearable device 200 may apply a higher weight value to a biometric signal obtained from the second photo-sensor array 330b than to a biometric signal obtained from the first photo-sensor array 330a. For example, the wearable device 200 may apply (e.g., multiply) a third weight value (e.g., a value equal to or greater than 1) to a size (e.g., the strength) of a biometric signal obtained from the second photo-sensor array 330b, and/or apply (e.g., multiply) a fourth weight value (e.g., a value smaller than 1) to a size (e.g., the strength) of a biometric signal obtained from the first photo-sensor array 330a, so as to identify biometric information of the wearer 503, based on a result of the application of the third weight value and/or the fourth weight value. As another example, the wearable device 200 may preferentially activate only the second photo-sensor array 330b (e.g., may output light only through the light emitting units of the second photo-sensor array 330b, or may receive light only through the light receiving units of the second photo-sensor array 330b or output a biometric signal), and then may activate the first photo-sensor array 330a as necessary (e.g., may operate only the light emitting unit and/or the light receiving unit of the first photo-sensor array 330a).

The arrangement of the photo-sensor arrays described with reference to FIG. 5C or FIG. 5D corresponds to an example, and the photo-sensor arrays may be provided in various arrangements. For example, the first photo-sensor array 330a or the second photo-sensor array 330b may be omitted. As another example, a photo-sensor array (e.g., the second photo-sensor array 330b in FIG. 5C) facing the second direction ③ and a photo-sensor array (e.g., the second photo-sensor array 330b in FIG. 5D facing the third direction (4) may be both arranged at the wearing member 320. In this case, a photo-sensor array (e.g., the first photo-sensor array 330a in FIG. 5C or FIG. 5D) facing the first direction ② may be omitted. As another example, one or more photo-sensor arrays (not illustrated) facing one or more directions different from the first direction to the third direction may be further arranged.

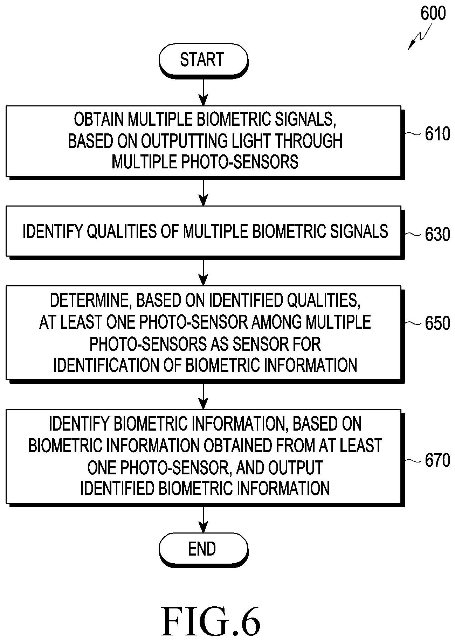

FIG. 6 is a flowchart 600 of a method of determining, by a wearable device (e.g., the wearable device 200 in FIG. 2A), a photo-sensor (e.g., the photo-sensor 201 in FIG. 2A) for identification of biometric information according to various embodiments.

According to various embodiments, the wearable device 200 may obtain a plurality of biometric signals, based on outputting light through a plurality of photo-sensors, in operation 610. For example, when the wearable device 200 includes a single photo-sensor array (e.g., the photo-sensor array 330 in FIG. 5A or 5B) in one or each of a pair of wearing members (e.g., the wearable devices 320 in FIG. 3) (e.g., as illustrated in FIG. 5A or FIG. 5B), the plurality of photo-sensors may indicate photo-sensors configuring a single photo-sensor array included in one or each of the pair of wearing members 320. As another example, when the wearable device 200 includes a plurality of photo-sensor arrays (e.g., the photo-sensor arrays 330a and 330b in FIG. 5C or 5D) in one or each of a pair of wearing members (e.g., the wearable devices 320 in FIG. 3) (e.g., as illustrated in FIG. 5C or FIG. 5D), the plurality of photo-sensors may indicate photo-sensors configuring a plurality of photo-sensor arrays included in one or each of the pair of wearing members 320. According to various embodiments, the wearable device 200 may activate all or some of the plurality of photo-sensors to output light through a light emitting unit (e.g., the light emitting unit 403 in FIG. 4) of each of the activated photo-sensors, and receive light through a light receiving unit (e.g., the light receiving unit 405 in FIG. 4) of each of the activated photo-sensors. According to various embodiments, the wearable device 200 may sequentially activate all or some of the plurality of photo-sensors. For example, referring to FIG. 5A together, the wearable device 200 may activate the first light emitting unit 403a and the first light receiving unit 405a, and then activate the second light emitting unit 403b and the second light receiving unit 405b adjacent to the first light emitting unit 403a and the first light receiving unit 405a. As another example, the wearable device 200 may activate the first light emitting unit 403a and the first light receiving unit 405a, then deactivate the first light receiving unit 405a, and activate the second light receiving unit 405b adjacent to the first light emitting unit 403a, so as to receive, through the second light receiving unit 405b, light received based on the light output through the first light emitting unit 403a. As yet another example, the wearable device 200 may activate the first light emitting unit 403a and the second light receiving unit 405b, then deactivate the first light emitting unit 403a, and activate the second light emitting unit 403b adjacent to the second light receiving unit 405b, so as to receive, through the second light receiving unit 405b, light received based on the light output through the second light emitting unit 403b. As yet another example, the wearable device 200 may activate together the second light receiving unit 405b and the first and second light emitting units 403a and 403b adjacent to the second light receiving unit 405b, and may also activate the third light receiving unit 405c and the second and third light emitting units 403b, and 403c adjacent to the third light receiving unit 405c. As yet another example, referring to FIG. 5B together, the wearable device 200 may activate the light emitting units and the light receiving units within a range providing a high possibility of contact of a wearer's body (e.g., an ear), for example, the range of about 7 cm to 10 cm, and then may also further and sequentially activate light emitting units and light receiving units adjacent to the range. The method of activating light emitting units and/or light receiving units described above corresponds to an example, and light emitting units and/or light receiving units may be activated in various other orders. According to various embodiments, the wearable device 200 may sequentially or simultaneously output at least one light (e.g., infrared rays, red light, green light, or blue light) through each of activated light emitting units (e.g., the light emitting unit 403 in FIG. 4), and may obtain (e.g., receive) at least one biometric signal (e.g., a biometric signal corresponding to an infrared ray, a biometric signal corresponding to red light, a biometric signal corresponding to green light, or a biometric signal corresponding to blue light) corresponding to the at least one output light, from each of activated light receiving units (e.g., the light receiving unit 405 in FIG. 4).

According to various embodiments, in operation 630, the wearable device 200 may identify qualities of the plurality of biometric signals. For example, the identified quality of a biometric signal may be the SNR of the biometric signal. For example, when at least one biometric signal (e.g., a biometric signal corresponding to an infrared ray, a biometric signal corresponding to red light, a biometric signal corresponding to green light, or a biometric signal corresponding to blue light) corresponding to at least one light is obtained by the wearable device 200 from each of the plurality of photo-sensors, the wearable device 200 may identify the quality (e.g., SNR) of each of the at least one biometric signal (e.g., a biometric signal corresponding to an infrared ray, a biometric signal corresponding to red light, a biometric signal corresponding to green light, or a biometric signal corresponding to blue light).

According to various embodiments, the wearable device 200 may determine, based on the identified qualities, at least one photo-sensor among the multiple photo-sensors as a sensor for identification of biometric information in operation 650. For example, the wearable device 200 may determine, as a sensor for identification of biometric information, at least one photo-sensor (e.g., a photo-sensor which provides a relatively high quality of biometric signal, and the number of which is a designated number or greater, or the designated number or smaller) which has output a relatively high quality of biometric signal. As another example, the wearable device 200 may determine, as sensors for identification of biometric information, photo-sensors providing biometric signal qualities equal to or greater than a predetermined threshold value. As another example, when at least one biometric signal (e.g., a biometric signal corresponding to an infrared ray, a biometric signal corresponding to red light, a biometric signal corresponding to green light, or a biometric signal corresponding to blue light) corresponding to at least one light is obtained by the wearable device 200 from each of the plurality of photo-sensors, the wearable device 200 may identify the quality (e.g., SNR) of each of the at least one biometric signal (e.g., a biometric signal corresponding to an infrared ray, a biometric signal corresponding to red light, a biometric signal corresponding to green light, or a biometric signal corresponding to blue light), and identify the average value of the identified qualities. For example, in a case where the wearable device 200 outputs green light, an infrared ray, and red light, and receives biometric signals corresponding to same, if the SNR of a biometric signal corresponding to the green light is 27.594, the SNR of a biometric signal corresponding to the infrared ray is 22.156, and the SNR of a biometric signal corresponding to the red light is 18.482, the wearable device may identify that the average value is 68.232. The wearable device 200 may individually identify the average value of biometric signal qualities with respect to each of the plurality of photo-sensors, and compare the average values so as to determine, as a photo-sensor for identification of biometric information, at least one photo-sensor, which has a relatively high average value, and the number of which is a designated number or greater, or the designated number or smaller, or at least one photo-sensor having an average value having a predetermined threshold value or higher.

According to various embodiments, the wearable device 200 may identify biometric information, based on a biometric signal obtained from the at least one photo-sensor, and output the identified biometric information in operation 670. For example, the wearable device 200 may activate the determined at least one photo-sensor, output light to a wearer's body (e.g., skin), then receive light obtained through reflection by the wearer's body (e.g., skin), and identify the wearer's biometric information, based on a biometric signal obtained from the at least one photo-sensor through the reception. For example, the wearer's biometric information may include information on a heart rate (or a pulse rate), an oxygen saturation level of blood, stress, or a blood pressure. As another example, the wearable device 200 may identify the wearer's biometric information, based on at least one biometric signal obtained from the determined at least one photo-sensor among the plurality of biometric signals obtained in operation 610. According to various embodiments, the wearable device 200 may visually output (e.g., display) the identified biometric information through a display (e.g., the display 203 in FIGS. 2A and 2B), or may also output (e.g., transmit) the identified biometric information to an external electronic device (e.g., the external electronic device 207 in FIG. 2B). According to various embodiments, the wearable device 200 may activate a photo-sensor different from the determined at least one photo-sensor together, and this process will be described in more detail with reference to the drawings described below.

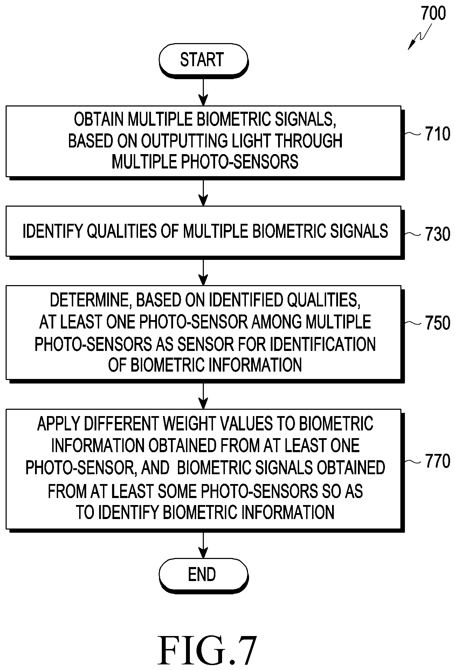

FIG. 7 is a flowchart 700 of a method of identifying, by a wearable device (e.g., the wearable device 200 in FIG. 2A), biometric information, based on a weight value according to various embodiments. A description overlapping with the above description given with reference to the drawings will be omitted.

According to various embodiments, the wearable device 200 may obtain a plurality of biometric signals, based on outputting light through a plurality of photo-sensors, in operation 710.

According to various embodiments, in operation 730, the wearable device 200 may identify qualities of the plurality of biometric signals.

According to various embodiments, the wearable device 200 may determine, based on the identified qualities, at least one photo-sensor among the plurality of photo-sensors as a sensor for identification of biometric information in operation 750.

According to various embodiments, the wearable device 200 may apply different weight values to biometric information obtained from the at least one photo-sensor, and a biometric signal obtained from at least one first photo-sensor so as to identify biometric information in operation 770. The at least one first photo-sensor may indicate all or some of the remaining photo-sensors other than the at least one photo-sensor among the plurality of photo-sensors. According to various embodiments, the wearable device 200 may activate the determined at least one photo-sensor and the at least one first photo-sensor, output light to a wearer's body (e.g., skin), and then receive light obtained through reflection by the wearer's body (e.g., skin), so as to obtain biometric signals from the at least one photo-sensor and the at least one first photo-sensor, respectively. The wearable device 200 may apply a higher weight value to a biometric signal obtained from the determined at least one photo-sensor than to a biometric signal obtained from the at least one first photo-sensor. For example, the wearable device 200 may apply (e.g., multiply) a first weight value (e.g., a value equal to or greater than 1) to size (e.g., the strength) of biometric signal obtained from the at least one photo-sensor, and/or apply (e.g., multiply) a second weight value (e.g., a value smaller than 1) to size (e.g., the strength) of biometric signal obtained from the at least one first photo-sensor, so as to identify biometric information of a wearer (e.g., the wearer 503 in FIG. 5C), based on a result of the application of the first weight value and/or the second weight value.

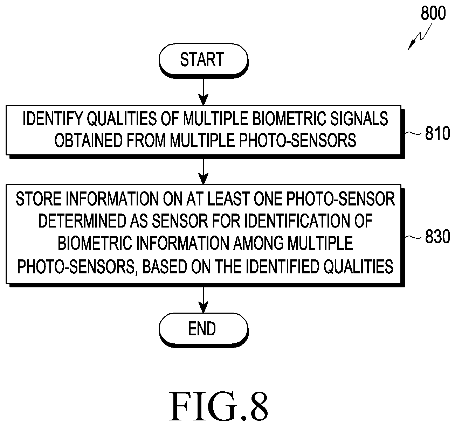

FIG. 8 is a flowchart 800 of a method of storing, by a wearable device (e.g., the wearable device 200 in FIGS. 2A and 2B), information on a photo-sensor determined as a sensor for identification of biometric information according to various embodiments. A description overlapping with the above description given with reference to the drawings will be omitted.

According to various embodiments, in operation 810, the wearable device 200 may identify qualities of the plurality of biometric signals obtained from a plurality of photo-sensors.

According to various embodiments, the wearable device 200 may store information on at least one photo-sensor determined as a sensor for identification of biometric information among the plurality of photo-sensors, based on the identified qualities, in operation 830. According to various embodiments , the information on the at least one photo-sensor may be stored in at least one of a memory (e.g., the memory 130 in FIG. 1) of the wearable device 200 or a memory (not illustrated) of an external electronic device (e.g., the external electronic device 207 in FIG. 2B).

According to various embodiments, the information on the at least one photo-sensor may be stored to correspond to a wearer. For example, if the wearer is identified to be a first user, information on at least one photo-sensor determined when the wearable device 200 is worn on the first user may be stored in association with identification information of the first user, and if the wearer is identified to be a second user, information on at least one photo-sensor determined when the wearable device 200 is worn on the second user may be stored in association with identification information of the second user. The identification information of the wearer may include, for example, information input to the wearable device 200 or the external electronic device 207 by the wearer at the time of operation of the wearable device 200. According to various embodiments, thereafter, when the identification information of the wearer is input to the wearable device 200 or the external electronic device 207 at the time of operation of the wearable device 200, the wearable device 200 may identify information on at least one photo-sensor, which is stored in association with the identification information of the wearer, and start an operation for identifying biometric information of the wearer, by using the determined at least one photo-sensor.

According to various embodiments, the information on the at least one photo-sensor may also be stored regardless of the wearer (e.g., may be stored with no relation to identification information of a user). For example, when information on at least one photo-sensor is stored, and then an input to start an operation of the wearable device 200 is received, the recently stored information on the at least one photo-sensor may be identified, and an operation for identifying biometric information of the wearer may be started by using the determined at least one photo-sensor. According to various embodiments, because the current wearer can be different from the recent wearer, the wearable device 200 may identify the quality of a biometric signal obtained from the determined at least one photo-sensor, and maintain the at least one photo-sensor as a sensor for identification of biometric information of the wearer, or change same into other sensors.

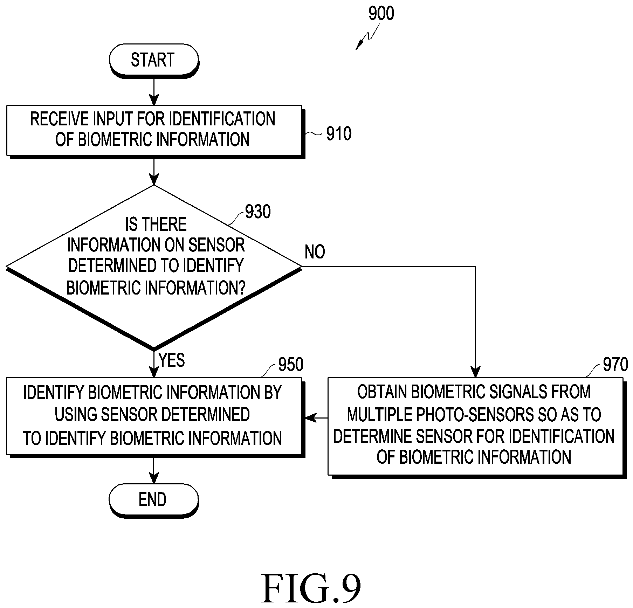

FIG. 9 is a flowchart 900 of an operation related to whether a photo-sensor for identification of biometric information is determined by a wearable device (e.g., the wearable device 200 in FIG. 2A), according to various embodiments. A description overlapping with the above description given with reference to the drawings will be omitted.

According to various embodiments, in operation 910, the wearable device 200 may obtain an input for identification of biometric information. For example, the wearable device 200 may obtain an input (e.g., an input to turn on the power of the wearable device 200) through an input module (e.g., the input module 150 in FIG. 1) of the wearable device 200. As another example, the wearable device 200 may use a sensor module (e.g., the sensor module 176 in FIG. 1) (e.g., a proximity sensor) to sense that the wearable device 200 is worn, thereby receiving information on the wearing of the wearable device 200, as an input for identification of biometric information. As another example, when an input is received through various input modules (e.g., a display) of an external electronic device (e.g., the external electronic device 207 in FIG. 2B), the wearable device 200 may obtain an input for identification of biometric information from the external electronic device 207 by a wire or wirelessly. For example, the input of the operation 901 may indicate an input to start the operation of the wearable device 200. As another example, the wearable device 200 may obtain an input to select a measurement mode (e.g., exercise, meditation, sensing of a heart problem, measuring of an oxygen saturation level, or measuring of stress) from an input module (e.g., the input module 150 in FIG. 1) or the external electronic device 207 by a wire or wirelessly.

According to various embodiments, in operation 930, the wearable device 200 may identify whether there is information on a sensor determined to identify biometric information. For example, the wearable device 200 may identify whether information on at least one photo-sensor determined as a sensor for identification of biometric information exists in a memory (e.g., the memory 130 in FIG. 1). For example, if identification information of a wearer is included in the input of operation 910, the wearable device 200 may identify whether there is information on at least one photo-sensor corresponding to the identification information of the wearer. As another example, if an input to select a measurement mode is received, the wearable device may identify whether there is information on at least one photo-sensor corresponding to the selected measurement mode.

According to various embodiments, based on identifying that there is information on a sensor determined to identify biometric information (e.g., based on identifying that there is information on at least one photo-sensor associated with the identification information of the wearer or the selected measurement mode), the wearable device 200 may identify the biometric information by using the sensor determined to identify biometric information in operation 950.

According to various embodiments, based on not identifying that there is information on a sensor determined to identify biometric information (e.g., based on determining that information on at least one photo-sensor is not stored in association with the identification information of the wearer or the selected measurement mode, or at least one photo-sensor for identification of biometric information has not been determined), the wearable device 200 may obtain biometric signals from a plurality of photo-sensors so as to determine a sensor for identification of biometric information in operation 970. For example, the wearable device 200 may sequentially or simultaneously activate all or some of the plurality of photo-sensors (e.g., a plurality of light emitting units and a plurality of light receiving units), and determine a sensor for identification of biometric information, based on the quality of a biometric signal obtained to correspond to received light. According to various embodiments, the wearable device 200 may perform the operation 970, and then identify biometric information by using the sensor determined to identify biometric information (operation 950).

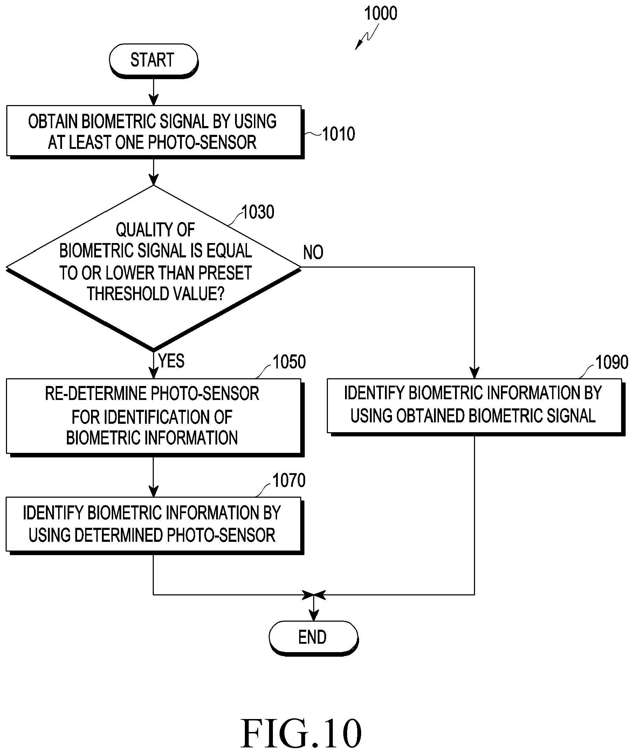

FIG. 10 is a flowchart 1000 of a method of re-determining, by a wearable device (e.g., the wearable device 200 in FIG. 2A), a photo-sensor for identification of biometric information according to various embodiments. A description overlapping with the above description given with reference to the drawings will be omitted.

According to various embodiments, in operation 1010, the wearable device 200 may obtain a biometric signal by using at least one photo-sensor. For example, the used at least one photo-sensor may be a sensor determined and stored to identify biometric information, based on the quality of a biometric signal, and may be at least one photo-sensor corresponding to identification information of the current wearer.

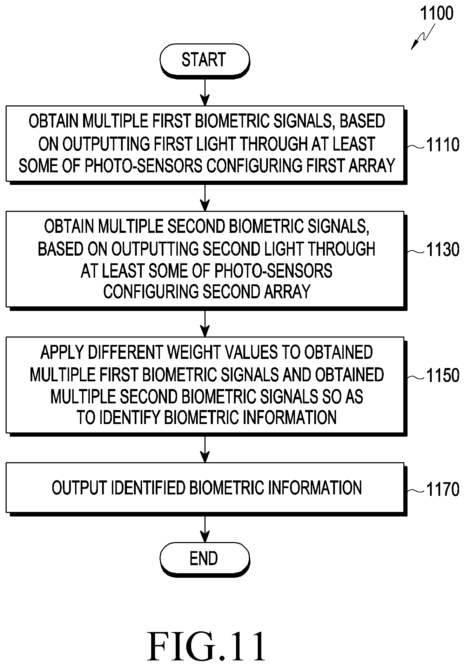

According to various embodiments, in operation 1030, the wearable device 200 may identify whether the quality of the biometric signal is equal to or lower than a preset threshold value. For example, the wearable device 200 may obtain a biometric signal from the at least one photo-sensor, identify the quality of the obtained biometric signal, and identify whether the identified quality is equal to or lower than a preset threshold value. According to various embodiments, the operation 1030 may be performed in an operation (e.g., the operation 670 in FIG. 6, the operation 770 in FIG. 7, the operation 950 in FIG. 9, or the operation 1150 in FIG. 11) of identifying biometric information, described with reference to the drawings in the disclosure, or may be performed before or after an operation (e.g., the operation 670 in FIG. 6, the operation 770 in FIG. 7, the operation 950 in FIG. 9, or the operation 1150 in FIG. 11) of identifying biometric information.

According to various embodiments, based on identifying that the quality of the biometric signal is equal to or lower than the preset threshold value, the wearable device 200 may re-determine a photo-sensor for identification of biometric information in operation 1050. For example, the wearer of the wearable device 200 has been changed, or for example, the wearable device 200 may sequentially or simultaneously activate all or some of a plurality of photo-sensors, and re-determine a sensor for identification of biometric information, based on the quality of a biometric signal obtained to correspond to received light. According to various embodiments, based on identifying that the quality of the biometric signal is equal to or lower than the preset threshold value, the wearable device 200 may output, through a display (e.g., the display 203 in FIG. 2A and/or a display of the external electronic device 207 in FIG. 2B), a message which requests a user to put the wearable device 200 on again. For example, if there is a foreign object (e.g., a hair) between a user's body and the wearable device 200, a message which requests removing of the foreign object and putting the wearable device on again may be displayed through a display (e.g., the display 203 in FIG. 2A and/or a display of the external electronic device 207 in FIG. 2B). According to various embodiments, based on identifying that the quality of the biometric signal is equal to or lower than the preset threshold value, the wearable device 200 may also output, through a voice output device (e.g., a speaker), a message (e.g., a voice message) which requests the user to put the wearable device 200 on again. When the user has put the wearable device 200 on again, the wearable device 200 may re-determine a photo-sensor for identification of biometric information. For example, the wearable device 200 may output a message which requests putting the wearable device 200 on again, and then, based on being sensed by using a sensor module (e.g., the sensor module 176 in FIG. 1) (e.g., a proximity sensor) that the wearable device 200 is worn, the wearable device 200 may identify that the wearable device has been put on again by the user.

According to various embodiments, in operation 1070, the wearable device 200 may identify biometric information by using the determined photo-sensor. For example, the wearable device 200 may obtain a biometric signal by using the photo-sensor re-determined in operation 1050, and identify biometric information, based on the obtained biometric signal.

According to various embodiments, based on identifying that the quality of the biometric signal is not equal to or lower than the preset threshold value, the wearable device 200 may identify biometric information by using the obtained biometric signal in operation 1090. For example, the wearable device 200 may identify biometric information of the wearer by using the biometric signal obtained in operation 1010. As another example, the wearable device 200 may identify the wearer's biometric information, based on a biometric signal newly obtained by using the at least one photo-sensor used in operation 1010.

FIG. 11 is a flowchart 1100 of a method of applying, by the wearable device 200, a weight value to a plurality of photo-sensor arrays (e.g., the first photo-sensor array 330a and the second photo-sensor array 330b in FIG. 5C or FIG. 5D) so as to identify biometric information according to various embodiments.

According to various embodiments, in operation 1110, the wearable device 200 may obtain a plurality of first biometric signals, based on outputting first light through at least some of photo-sensors configuring a first array (e.g., the first photo-sensor array 330a in FIG. 5C or FIG. 5D). For example, the first array (e.g., the first photo-sensor array 330a) may be disposed at a wearing member (e.g., the wearing member 320 in FIG. 3) to face a first direction (e.g., the direction ② in FIG. 5C or FIG. 5D). For example, the first light may be output in the first direction (e.g., the direction ② in FIG. 5C or FIG. 5D). For example, the plurality of first biometric signals may correspond to light received by all or some light receiving units of the photo-sensors configuring the first array (e.g., the first photo-sensor array 330a in FIG. 5C or FIG. 5D) through reflection of, by a wearer's body (e.g., skin), the first light output in the first direction (e.g., the direction ② in FIG. 5C or FIG. 5D).

According to various embodiments, in operation 1130, the wearable device 200 may obtain a plurality of second biometric signals, based on outputting second light through at least some of photo-sensors configuring a second array (e.g., the second photo-sensor array 330b in FIG. 5C or FIG. 5D). For example, the second array (e.g., the second photo-sensor array 330b) may be disposed at a wearing member (e.g., the wearing member 320 in FIG. 3) to face a second direction (e.g., the direction ③ in FIG. 5C or FIG. 5D) or a third direction (e.g., the direction (4) in FIG. 5C or FIG. 5D). For example, the second light may be output in the second direction (e.g., the direction ③ in FIG. 5C or FIG. 5D) or the third direction (e.g., the direction (4) in FIG. 5C or FIG. 5D). For example, the plurality of second biometric signals may correspond to light received by all or some light receiving units of the photo-sensors configuring the second array (e.g., the second photo-sensor array 330b in FIG. 5C or FIG. 5D) through reflection of, by a wearer's body (e.g., skin), the second light output in the second direction (e.g., the direction ③ in FIG. 5C or FIG. 5D) or the third direction (e.g., the direction (4) in FIG. 5C or FIG. 5D). According to various embodiments, the photo-sensors configuring the second photo-sensor array may be arranged at an external wearable device (not illustrated) (e.g., a device having a watch type, a ring type, or a patch type). For example, the external wearable device may include the photo-sensors configuring the second photo-sensor array. The external wearable device may obtain a plurality of second biometric signals, based on outputting the second light through at least some of the photo-sensors configuring the second photo-sensor array, and transmit information on the obtained biometric signals to the wearable device 200. The wearable device 200 may obtain information on biometric signals transmitted by the wearable device from the external wearable device.

According to various embodiments, in operation 1150, the wearable device 200 may apply different weight values to the obtained a plurality of first biometric signals and the obtained a plurality of second biometric signals so as to identify biometric information. For example, the wearable device 200 may apply (e.g., multiply) a third weight value (e.g., a value equal to or greater than 1) to sizes (e.g., the strength) of the plurality of second biometric signals, and/or apply (e.g., multiply) a fourth weight value (e.g., a value smaller than 1) to sizes (e.g., the strength) of the plurality of first biometric signals, so as to identify biometric information of the wearer 503, based on a result of the application of the third weight value and/or the fourth weight value.

According to various embodiments, in operation 1170, the wearable device 200 may output the identified biometric information. For example, the wearable device 200 may visually output (e.g., display) the identified biometric information through a display (e.g., the display 203 in FIGS. 2A and 2B), or may also output (e.g., transmit) the identified biometric information to an external electronic device (e.g., the external electronic device 207 in FIG. 2B).

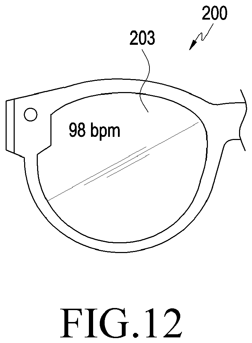

FIG. 12 illustrates an example of a screen showing biometric information which may be displayed through the display 203 of a wearable device (e.g., the wearable device 200 in FIG. 2A) according to various embodiments.

According to various embodiments, information indicating identified biometric information of a wearer may be displayed on the display 203 of the wearable device 200. For example, the information which may be displayed, that is, the biometric information of the wearer, may include information on a heart rate (or a pulse rate), an oxygen saturation level of blood, stress, or a blood pressure.

For example, referring to FIG. 12, the information indicating the biometric information of the wearer may be displayed by using a specific value through the display 203. For example, information on a heart rate (or a pulse rate) may be displayed by using a specific value (e.g., 98 bpm). In addition, information on an oxygen saturation level of blood, stress, or a blood pressure may be displayed by using a specific value.

As another example, although not illustrated, various information which can be predicted from the identified biometric information may be displayed through the display 203. For example, when it is identified that the wearer's heart rate (or pulse rate) is beyond a normal range, a health problem of the wearer may be predicted, and a text or image indicating the health problem of the wearer may be displayed.

In addition to the above examples, various information which shows at least one piece of biometric information of the wearer or is related to biometric information may be displayed through the display 203 in a text and/or image type.

According to various embodiments, a wearable device (e.g., the wearable device 200 in FIG. 2A) may include a housing (e.g., the housing 310 in FIG. 3), a plurality of photo-sensors (e.g., the photo-sensor 201 in FIG. 2A) that are arranged on the housing or arranged to be exposed outside through the housing, and configure at least one array, and at least one processor (e.g., the processor 120 in FIG. 2A), wherein the at least one processor is configured to obtain a plurality of biometric signals, based on outputting light through the plurality of photo-sensors, identify qualities of the obtained a plurality of biometric signals, determine, based on the identified qualities, at least one photo-sensor among the plurality of photo-sensors as a sensor for identification of biometric information, and identify biometric information, based on a biometric signal obtained from the at least one photo-sensor, and output the identified biometric information.

According to various embodiments, the at least one processor may be further configured to, based on determining the at least one photo-sensor as the sensor for identification of biometric information, control the at least one photo-sensor among the plurality of photo-sensors to output at least one light.

According to various embodiments, the at least one processor may be further configured to identify a biometric signal obtained from the at least one photo-sensor, and a biometric signal obtained from at least one first photo-sensor among the plurality of photo-sensors, the at least one first photo-sensor being different from the at least one photo-sensor, and apply a first weight value to the biometric signal obtained from the at least one photo-sensor, and apply a second weight value to the biometric signal obtained from the at least one first photo-sensor, and the first weight value may be greater than the second weight value.

According to various embodiments, the wearable device may further include a memory (e.g., the memory 130 in FIG. 2A), and the at least one processor may be further configured to store, in the memory, information on the at least one photo-sensor determined as the sensor for identification of biometric information.

According to various embodiments, the at least one processor may be configured to identify information on a wearer of the wearable device, and store the information on the at least one photo-sensor determined as the sensor for identification of biometric information in association with the information on the wearer.

According to various embodiments, the at least one processor may be configured to obtain an input for identification of the biometric information, based on the obtaining of the input, identify the information on the at least one photo-sensor, which is stored in the memory, and identify the biometric information, based on a biometric signal obtained from the at least one photo-sensor, based on the identified information.

According to various embodiments, the plurality of photo-sensors may configure a first array (e.g., the first photo-sensor array 330a in FIG. 5C) and a second array (e.g., the second photo-sensor array 330b in FIG. 5C), the first array may be disposed to face a first direction, and the second array may be disposed to face a second direction or a third direction which is different from the first direction.

According to various embodiments, the at least one processor may be further configured to apply a third weight value to a biometric signal obtained from at least one second sensor configuring the first array, and apply a fourth weight value to a biometric signal obtained from at least one third sensor configuring the second array, and the fourth weight value may be greater than the third weight value.

According to various embodiments, the at least one processor may be further configured to identify quality of a biometric signal obtained from the at least one photo-sensor, and based on identifying that the quality of the biometric signal obtained from the at least one photo-sensor is lower than a preset threshold value, re-determine the sensor for identification of biometric information.

According to various embodiments, a method for controlling a wearable device may include obtaining a plurality of biometric signals, based on outputting light through a plurality of photo-sensors of the wearable device, the plurality of photo-sensors being arranged on a housing of the wearable device or arranged to be exposed outside through the housing, identifying qualities of the obtained a plurality of biometric signals, determining, based on the identified qualities, at least one photo-sensor among the plurality of photo-sensors as a sensor for identification of biometric information, and identifying biometric information, based on a biometric signal obtained from the at least one photo-sensor, and outputting the identified biometric information.

According to various embodiments, the method for controlling the wearable device may further include, based on determining the at least one photo-sensor as the sensor for identification of biometric information, outputting at least one light through the at least one photo-sensor among the plurality of photo-sensors.

According to various embodiments, the method for controlling the wearable device may further include identifying a biometric signal obtained from the at least one photo-sensor, and a biometric signal obtained from at least one first photo-sensor among the plurality of photo-sensors, the at least one first photo-sensor being different from the at least one photo-sensor, and applying a first weight value to the biometric signal obtained from the at least one photo-sensor, and apply a second weight value to the biometric signal obtained from the at least one first photo-sensor, and the first weight value may be greater than the second weight value.

According to various embodiments, the method for controlling the wearable device may further include storing, in a memory of the wearable device, information on the at least one photo-sensor determined as the sensor for identification of biometric information.

According to various embodiments, the storing of the information on the at least one photo-sensor determined as the sensor for identification of biometric information in the memory of the wearable device may include identifying information on a wearer of the wearable device and storing the information on the at least one photo-sensor determined as the sensor for identification of biometric information in association with the information on the wearer.

According to various embodiments, the identifying of the biometric information, based on the biometric signal obtained from the at least one photo-sensor, and the outputting of the identified biometric information may include obtaining an input for identification of the biometric information, identifying, based on the obtaining of the input, the information on the at least one photo-sensor, which is stored in the memory, and identifying the biometric information, based on a biometric signal obtained from the at least one photo-sensor, based on the identified information.

According to various embodiments, the plurality of photo-sensors may configure a first array and a second array, the first array may be disposed to face a first direction, the second array may be disposed to face a second direction or a third direction which is different from the first direction, the method for controlling the wearable device may further include applying a third weight value to a biometric signal obtained from at least one second sensor configuring the first array, and applying a fourth weight value to a biometric signal obtained from at least one third sensor configuring the second array, and the fourth weight value may be greater than the third weight value.

According to various embodiments, the method for controlling the wearable device may further include identifying quality of a biometric signal obtained from the at least one photo-sensor, and based on identifying that the quality of the biometric signal obtained from the at least one photo-sensor is lower than a preset threshold value, re-determining the sensor for identification of biometric information.

According to various embodiments, a wearable device may include a housing, a plurality of photo-sensors arranged to be exposed outside through the housing, the plurality of photo-sensors configuring a first array disposed to face a first direction, and a second array disposed to face a second direction different from the first direction, and at least one processor, wherein the at least one processor is configured to obtain a plurality of first biometric signals, based on outputting first light through at least some of photo-sensors configuring the first array, obtain a plurality of second biometric signals, based on outputting second light through at least some of photo-sensors configuring the second array, apply different weight values to the obtained a plurality of first biometric signals and the obtained a plurality of second biometric signals so as to identify biometric information, and output the identified biometric information.

According to various embodiments, the at least one processor may be further configured to identify qualities of the obtained a plurality of first biometric signals and qualities of the obtained a plurality of second signals, and identify at least some of the plurality of sensors as sensors for identification of biometric information, based on the identified qualities of the a plurality of first biometric signals and the identified qualities of the plurality of second signals.

According to various embodiments, the wearable device may further include a memory, and the at least one processor may be further configured to store, in the memory, information on the at least some of the plurality of sensors, which are identified as the sensors for identification of biometric information.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., an internal memory 136 or an external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., a compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or a plurality of entities, and some of the plurality of entities may be separately disposed in different components. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device comprising:
a housing;
a plurality of photo-sensors that are arranged on the housing or arranged to be exposed outside through the housing, and configure at least one array; and
at least one processor,
wherein the at least one processor is configured to:
obtain a plurality of biometric signals, based on outputting light through the plurality of photo-sensors,
identify qualities of the obtained multiple biometric signals, determine, based on the identified qualities, at least one photo-sensor among the plurality of photo-sensors as a sensor for identification of biometric information,
identify biometric information, based on a biometric signal obtained from the at least one photo-sensor, and
output the identified biometric information.

2. The wearable device of claim 1, wherein the at least one processor is further configured to, based on determining the at least one photo-sensor as the sensor for identification of biometric information, control the at least one photo-sensor among the plurality of photo-sensors to output at least one light.

3. The wearable device of claim 1,
wherein the at least one processor is further configured to:
identify a biometric signal obtained from the at least one photo-sensor, and a biometric signal obtained from at least one first photo-sensor among the plurality of photo-sensors, the at least one first photo-sensor being different from the at least one photo-sensor, and
apply a first weight value to the biometric signal obtained from the at least one photo-sensor, and apply a second weight value to the biometric signal obtained from the at least one first photo-sensor, and
wherein the first weight value is greater than the second weight value.

4. The wearable device of claim 1, further comprising a memory,
wherein the at least one processor is further configured to store, in the memory, information on the at least one photo-sensor determined as the sensor for identification of biometric information.

5. The wearable device of claim 4, wherein the at least one processor is configured to:
identify information on a wearer of the wearable device, and
store the information on the at least one photo-sensor determined as the sensor for identification of biometric information in association with the information on the wearer.

6. The wearable device of claim 4, wherein the at least one processor is further configured to:
obtain an input for identification of the biometric information,
based on the obtaining of the input, identify the information on the at least one photo-sensor, which is stored in the memory, and
identify the biometric information, based on a biometric signal obtained from the at least one photo-sensor, based on the identified information.

7. The wearable device of claim 1,
wherein the plurality of photo-sensors configure a first array and a second array,
wherein the first array is disposed to face a first direction, and
wherein the second array is disposed to face a second direction or a third direction which is different from the first direction.

8. The wearable device of claim 7,
wherein the at least one processor is further configured to:
apply a third weight value to a biometric signal obtained from at least one second sensor configuring the first array, and
apply a fourth weight value to a biometric signal obtained from at least one third sensor configuring the second array, and
wherein the fourth weight value is greater than the third weight value.

9. The wearable device of claim 1, wherein the at least one processor is further configured to:
identify quality of a biometric signal obtained from the at least one photo-sensor, and
based on identifying that the quality of the biometric signal obtained from the at least one photo-sensor is lower than a preset threshold value, re-determine the sensor for identification of biometric information.

10. A method for controlling a wearable device, the method comprising:
obtaining a plurality of biometric signals, based on outputting light through multiple photo-sensors of the wearable device, the multiple photo-sensors being arranged on a housing of the wearable device or arranged to be exposed outside through the housing;
identifying qualities of the obtained multiple biometric signals;
determining, based on the identified qualities, at least one photo-sensor among the plurality of photo-sensors as a sensor for identification of biometric information;
identifying biometric information, based on a biometric signal obtained from the at least one photo-sensor; and
outputting the identified biometric information.

11. The method of claim 10, further comprising, based on determining the at least one photo-sensor as the sensor for identification of biometric information, outputting at least one light through the at least one photo-sensor among the plurality of photo-sensors.

12. The method of claim 10, further comprising:
identifying a biometric signal obtained from the at least one photo-sensor, and a biometric signal obtained from at least one first photo-sensor among the plurality of photo-sensors, the at least one first photo-sensor being different from the at least one photo-sensor;
applying a first weight value to the biometric signal obtained from the at least one photo-sensor; and
applying a second weight value to the biometric signal obtained from the at least one first photo-sensor,
wherein the first weight value is greater than the second weight value.

13. The method of claim 10, further comprising storing, in a memory of the wearable device, information on the at least one photo-sensor determined as the sensor for identification of biometric information.

14. The method of claim 13, wherein the storing of the information on the at least one photo-sensor determined as the sensor for identification of biometric information in the memory of the wearable device comprises:
identifying information on a wearer of the wearable device; and
storing the information on the at least one photo-sensor determined as the sensor for identification of biometric information in association with the information on the wearer.

15. The method of claim 13, wherein the identifying of the biometric information, based on the biometric signal obtained from the at least one photo-sensor, and the outputting of the identified biometric information comprises:
obtaining an input for identification of the biometric information;
based on the obtaining of the input, identifying the information on the at least one photo-sensor, which is stored in the memory; and
identifying the biometric information, based on a biometric signal obtained from the at least one photo-sensor, based on the identified information.
